# EUROPEAN PATENT SPECIFICATION

(11) **EP 3 483 172 B1**
(45) Date of publication and mention of the grant of the patent: **26.01.2022**
(21) Application number: 16907773.2
(22) Date of filing: 05.07.2016
(51) Int. Cl.: C07K 1/13, C07K 19/00, C09B 15/00, C09B 69/10, G01N 33/48, G01N 33/532, G01N 33/58

(54) **ACRIDINE-MARKER CONJUGATE AND PREPARATION METHOD THEREOF, AND CHEMILUMINESCENCE KIT**
ACRIDIN-MARKER-KONJUGAT UND HERSTELLUNGSVERFAHREN DAFÜR UND CHEMILUMINESZENZ-KIT
CONJUGUÉ ACRIDINE-MARQUEUR ET SON PROCÉDÉ DE PRÉPARATION, ET KIT DE CHIMIOLUMINESCENCE

(43) Date of publication of application: 15.05.2019
(73) Proprietor: Shenzhen Yhlo Biotech Co., Ltd, 518116 Shenzhen (CN)
(72) Inventor: QIAN, Chungen, 518116 Shenzhen (CN); XIAO, Chengyong, 518116 Shenzhen (CN); ZHU, Liang, 518116 Shenzhen (CN); LIU, Taoxu, 518116 Shenzhen (CN); XIA, Fuzhen, 518116 Shenzhen (CN)
(74) Representative: Mewburn Ellis LLP
(86) International application number: PCT/CN2016/088625
(87) International publication number: WO 2018/006268

(56) References cited:
- WO-A1-03/079010
- WO-A1-2011/051950
- WO-A2-2012/110833
- CN-A- 103 588 872
- CN-A- 103 792 346
- CN-A- 103 857 698
- CN-A- 104 961 801
- CN-A- 106 053 443
- JP-A- 2010 143 883
- US-A1- 2012 035 320
- YEONGRAN LEE ET AL: "Amphiphilic Helical Peptides Containing Two Acridine Moieties Display Picomolar Affinity toward HIV-1 RRE and TAR", ANGEWANDTE CHEMIE, INTERNATIONAL EDITION, vol. 47, no. 1, 1 January 2008 (2008-01-01), pages 134-137, XP055332535, DE ISSN: 1433-7851, DOI: 10.1002/anie.200703090
- TOSELAND CHRISTOPHER P: "Fluorescent labeling and modification of proteins", JOURNAL OF CHEMICAL BIOLOGY, SPRINGER BERLIN HEIDELBERG, BERLIN/HEIDELBERG, vol. 6, no. 3, 13 April 2013 (2013-04-13), pages 85-95, XP035342280, ISSN: 1864-6158, DOI: 10.1007/S12154-013-0094-5 [retrieved on 2013-04-13]
- CHRISTOPHER S THEILE ET AL: "Site-specific N-terminal labeling of proteins using sortase-mediated reactions", NATURE PROTOCOLS, vol. 8, no. 9, 29 August 2013 (2013-08-29) , pages 1800-1807, XP055310904, GB ISSN: 1754-2189, DOI: 10.1038/nprot.2013.102

## Description

### TECHNICAL FIELD

The present disclosure relates to the field of in vitro detection, in particular to an acridine-labelled conjugate, a method for preparing the same and a chemiluminescent kit.

### BACKGROUND

Chemiluminescent labeling immunoassay, also known as chemiluminescence immunoassay (CLIA), is an immunoassay in which an antigen, a hapten, or an antibody is directly labeled with a chemiluminescent agent. A chemiluminescent substance used for labeling includes substituted acridine, and depending on different substituents, the chemiluminescent substance is divided into two categories: acridinium ester (AE) and acridine sulfonamide, both of which are effective luminescent labels and under action of a start luminescent reagent (NaOH, H₂O₂), generates a luminescence, which is an intense direct luminescence that completed in one second and is a fast blinking luminescence.

Substituted acridine, when is used in immunoassay as a chemiluminescent label, allows a simple and rapid chemical reaction without a catalyst and detection of a small molecule antigen by competition and a macromolecule antigen by sandwich assay, and has low non-specific binding and background. Substituted acridine will not bring a reduced amount of luminescence generated when binding to a macromolecule, and thus sensitivity improves. In terms of luminescence mechanism, such compounds are characterized by: 1. during a luminescent reaction, before formation of an electronically excited intermediate, a non-luminescent substituent moiety bound to a acridine ring is detached from the acridine ring, i.e., a non-luminescent moiety is separated from a luminescent moiety, and thus a structure of substituent substantially has no effect on luminous efficiency of substituted acridine. 2. No catalyst is required for chemiluminescence of acridinium ester or acridine sulfonamide compounds, and such compounds are capable of luminescence in a dilute alkaline solution containing H₂O₂. Therefore, when applied in chemiluminescence assay, acridinium ester or acridine sulfonamide compounds have many advantages , mainly comprising: ① low background luminescence and high signal to noise ratio; ②less interference factors for luminescence reaction; ③ fast and focused release of light, high luminescent efficiency and luminescent intensity; ④ prone to bind to a protein without a decreased photon yield after binding; and ⑤stable labels (which can be stored for several months at 2 °C to 8 °C). Substituted acridine is therefore a very effective and a very good chemiluminescent label.

An acridine-labelled conjugate is a complex obtained by binding substituted acridine to a substance to be labelled (e.g. antibody, antigen). The acridine-labelled conjugate has a quality that is directly related to whether the chemiluminescent immunoassay succeeds or not, and thus is referred to as a key reagent. Toseland Christopher P ("Fluorescent labeling and modification of proteins", JOURNAL OF CHEMICAL BIOLOGY, SPRINGER BERLIN HEIOELBERG, BERLIN HEIOELBERG, vol. 6, no. 3, 13 April 2013 (2013-04-13)) disclosed methods for protein labelling, in particular labelling of the N-terminal amino group using an active ester, and the N-terminal labelling allows to separate the fluorescent label and the active site, wherein the label is an acridine loaded polyamino acid.

So far, carbodiimide crosslinking is used as a conventional method for preparing the acridine-labelled conjugate, wherein a carbodiimide crosslinking agent is used as a bridge for binding substituted acridine to a protein to be labelled. However, in the acridine-labelled conjugate prepared by the conventional method, substituted acridine is bound to the protein to be labelled by carbodiimide, and frequently interferes with an active site on the protein to be labelled, resulting in decreased activity of the acridine-labelled conjugate, and affecting sensitivity for the immunoassay.

### SUMMARY

Based on this, it is necessary to provide an acridine-labelled conjugate with relatively high activity, a method for preparing the same, and a chemiluminescent kit.

The present invention provides an acridine-labelled conjugate comprising a substituted acridine, a polyamino acid, and a protein to be labelled which are sequentially linked;
the polyamino acid is a polyamino acid containing a carboxyl group and an amino group, and the polyamino acid reacts, by the amino group therein, with the substituted acridine to form a chemical bond;
the protein to be labelled is a protein, a modified protein, a polypeptide or a modified polypeptide containing an amino group, and the amino group in the protein to be labelled reacts with the carboxyl group in the polyamino acid to form a -NH-CO- structure, thereby linking the polyamino acid and the protein to be labelled together; and
the substituted acridine is 10-methyl-acridine-9-N-succinimidyl ester formic acid (AE-NHS) and the polyamino acid is polylysine.

The invention also provides a method for preparing the acridine-labelled conjugate described above and a chemiluminescence kit for binding a protein to be labelled to form the acridine-labelled conjugate described as above.

Such an acridine-labelled conjugate comprises a substituted acridine, a polyamino acid, and a protein to be labelled which are sequentially linked, the polyamino acid reacts, by an amino group therein, with the substituted acridine to form a chemical bond, and the amino group in the protein to be labelled reacts with the carboxyl group in the polyamino acid to form a -NH-CO- structure, thereby linking the polyamino acid and the protein to be labelled together. Due to a relatively identified binding site, the substituted acridine is prevented from interfering with an active site in the protein to be labelled, and thus the acridine-labelled conjugate has relatively high activity. In addition, the polyamino acid allows an increased steric hindrance of the acridine-labelled conjugate, thereby increasing the sensitivity when using the acridine-labelled conjugate.

### DESCRIPTION OF DRAWINGS

FIG. 1 is a flowchart of a method for preparing an acridine-labelled conjugate according to an embodiment;
FIG. 2 is a scatter gram for results from detecting PTH antigen samples having a serial of concentrations in Test example 2.

### DETAILED DESCRIPTION

Specific embodiments of the present disclosure will be described in detail below with reference to drawings and specific examples. Numerous specific details are set forth in the description below in order to provide a thorough understanding of the disclosure. However, the present disclosure can be implemented in many other ways than those described herein, and those skilled in the art may make similar modifications without departing from the scope of the present disclosure, and thus the present disclosure is not limited by the specific embodiments disclosed below, but is defined by the appended claims.

An acridine-labelled conjugate comprises a substituted acridine, a polyamino acid, and a protein to be labelled which are sequentially linked.

The polyamino acid may be polylysine having a degree of polymerization greater than 25. Preferably, the polyamino acid is polylysine having a degree of polymerization ranging from 100 to 200.

The protein to be labelled is a protein, a modified protein, a polypeptide or a modified polypeptide containing an amino group, and the amino group in the protein to be labelled reacts with the carboxyl group in the polyamino acid to form a -NH-CO- structure, thereby linking the polyamino acid and the protein to be labelled together.

The protein to be labelled may be a protein or a polypeptide having an amino group per se, or may be a modified protein or a modified polypeptide into which an amino group is introduced by modification.

In this embodiment, the protein to be labelled is an antigen, a hapten or an antibody.

The acridine-labelled conjugate has following structural formula: 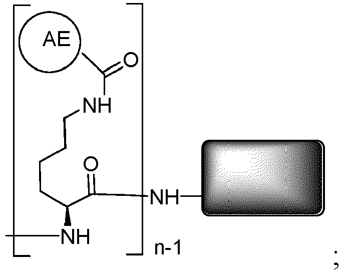 wherein, 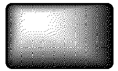 is the protein to be labeled having an amino residue, and n is an integer greater than 25, preferably within a range from 100 to 200.

Such an acridine-labelled conjugate comprises a substituted acridine, a polyamino acid, and a protein to be labelled which are sequentially linked, the polyamino acid reacts, by an amino group therein, with the substituted acridine to form a chemical bond, and the amino group in the protein to be labelled reacts with the carboxyl group in the polyamino acid to form a -NH-CO- structure, thereby linking the polyamino acid and the protein to be labelled together. Due to a relatively identified binding site, the substituted acridine is prevented from interfering with an active site in the protein to be labelled, and thus the acridine-labelled conjugate has relatively high activity.

In addition, the polyamino acid allows an increased steric hindrance of the acridine-labelled conjugate, thereby increasing sensitivity when using the acridine-labelled conjugate.

Compared with the conventional technology, in such an acridine-labelled conjugate containing the polyamino acid, the protein to be labelled is prevented from inactivation due to an effectively protected active site thereof, and the acridine-labelled conjugate is characterized by high stability, controllable amount of linkage, by sequentially linking the substituted acridine, the polyamino acid and the label to be labelled via the chemical bond.

Such acridine-labelled conjugate can be directly used for detection and quantitative analysis in a chemiluminescence immunoassay, and can solve disadvantages of acridine-labelled conjugate prepared by conventional carbodiimide cross-linking method, such as inactivation of a start material, poor signal.

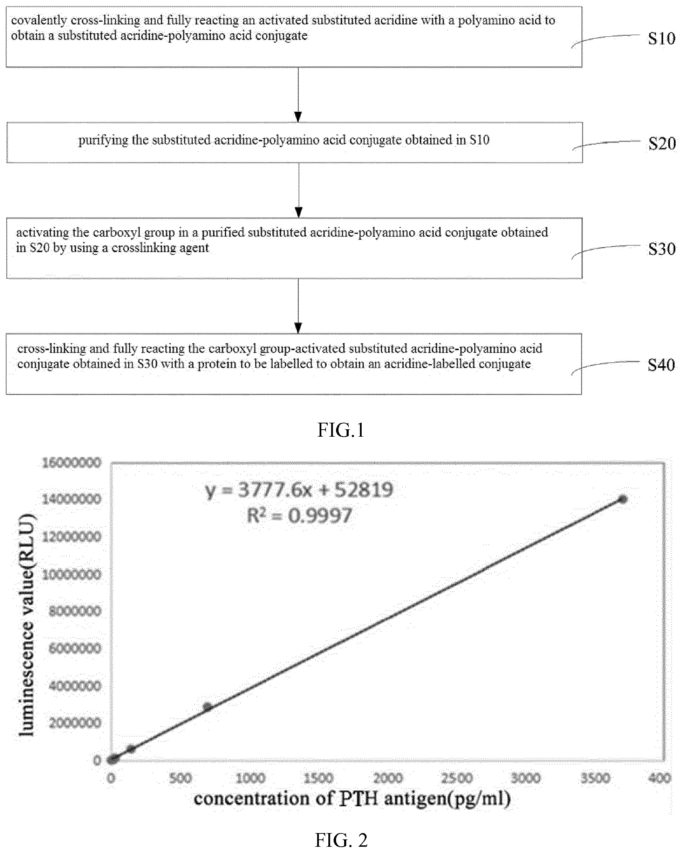

A method for preparing the acridine-labelled conjugate described above as shown in FIG. 1 comprises:
S10, covalently cross-linking and fully reacting an activated substituted acridine with a polyamino acid to obtain a substituted acridine-polyamino acid conjugate.

In covalently crosslinking the activated substituted acridine with the polyamino acid, a molar ratio of the activated substituted acridine to the polyamino acid ranges from 1:1 to 10000:1.

Preferably, the molar ratio of the substituted acridine to the polyamino acid ranges from 10:1 to 200:1.

The activated substituted acridine may be a carboxyl group-activated substituted acridine. The activated substituted acridine may be activated by carbodiimide and hydroxysuccinimide, and specific activation is as follows: adding and fully dissolving the substituted acridine in a buffer before adding EDC and NHS, reacting at 25°C for 10 min to obtain an activated substituted acridine, wherein the buffer is phosphate buffered saline, carbonate buffered saline, 2-(N-morpholino)ethanesulfonic acid (MES) buffer or piperazine-N,N'-bis(2-ethanesulfonic acid) (PIPES) buffer, and reaction is performed at a pH ranging from 4 to 10.

The polyamino acid may be polylysine having a degree of polymerization greater than 25. Preferably, the polyamino acid is polylysine having a degree of polymerization ranging from 100 to 200.

The substituted acridine is covalently cross-linked with the polyamino acid to obtain a substituted acridine-polyamino acid conjugate, as shown in following reaction scheme:

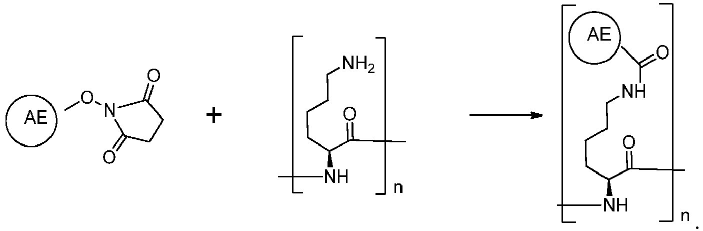

Specific reaction process for the above reaction scheme is as follows: adding and fully dissolving the polyamino acid in a buffer before adding excess acridinium ester, reacting at a temperature ranging from 4°C to 37°C for 0.5 h to 12 h, and after completion of the reaction, purifying to obtain an acridinium ester-polyamino acid conjugate, wherein the buffer is phosphate buffered saline, carbonate buffered saline, 2-(N-morpholino)ethanesulfonic acid (MES) buffer or piperazine-N,N'-bis(2-ethanesulfonic acid) (PIPES) buffer, and the reaction is performed at a pH ranging from 4 to 10.

Since the acridinium ester is present at an excess amount with respective to the polyamino acid, the amino group in the polyamino acid may be regarded as being completely exhausted and will not compete with the amino group in the protein to be labelled in a next stage, and thus no amino group in the acridinium ester-polyamino acid conjugate needs to be blocked.

S20, purifying the substituted acridine-polyamino acid conjugate obtained in S10.

Purifying the substituted acridine-polyamino acid conjugate may be one or more selected from the group consisting of ultrafiltration purification, desalting column purification, and dialysis purification.

S30, activating the carboxyl group in a purified substituted acridine-polyamino acid conjugate obtained in S20 by using a crosslinking agent.

The crosslinking agent comprises carbodiimide and hydroxysuccinimide, and a molar ratio of carbodiimide to the substituted acridine-polyamino acid conjugate ranges from 5:1 to 1000:1, and a molar ratio of carbodiimide to hydroxysuccinimide ranges from 10:1 to 1:20.

Preferably, carbodiimide is at least one selected from dicyclohexylcarbodiimide, 1-(3-dimethylamino propyl)-3-ethyl carbodiimide, and N,N'-diisopropyl carbodiimide.

Preferably, the molar ratio of carbodiimide to the substituted acridine-polyamino acid conjugate ranges from 20:1 to 500:1.

Preferably, hydroxysuccinimide is at least one selected from N-hydroxysuccinimide and N-hydroxy sulfo succinimide.

Preferably, the molar ratio of carbodiimide to hydroxysuccinimide ranges from 3:1 to 1:10.

The carboxyl group in the substituted acridine-polyamino acid conjugate is activated by using carbodiimide and hydroxysuccinimide, as shown in following reaction scheme: 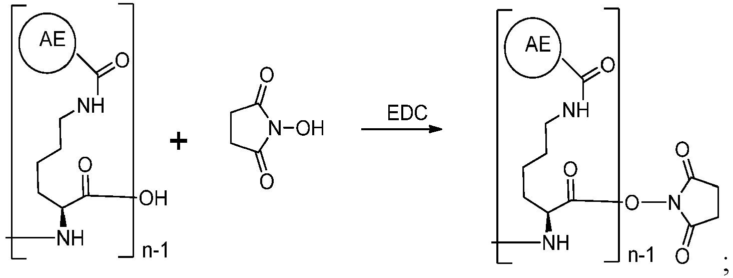 wherein n is an integer greater than 25, preferably within a range from 100 to 200.

Specific reaction process of the above reaction scheme is as follows: adding and fully dissolving the substituted acridine-polyamino acid conjugate in a buffer, before adding EDC and NHS, reacting at 25°C for 10 min to obtain an carboxyl group-activated substituted acridine-polyamino acid conjugate, wherein, the buffer is phosphate buffered saline, carbonate buffered saline, 2-(N-morpholino)ethanesulfonic acid (MES) buffer or piperazine-N,N'-bis(2-ethanesulfonic acid) (PIPES) buffer, and the reaction is performed at a pH ranging from 4 to 10.

Preferably, S30 further comprises: adding a mercapto compound (such as mercaptoethanol, mercapto threitol, cysteine, cysteine hydrochloride) for quenching activity of the cross-linking agent (or purifying for removal of the cross-linking agent) after the carboxyl group in the substituted acridine-polyamino acid conjugate is activated, so as to obtain a carboxyl group-activated substituted acridine-polyamino acid conjugate having a blocked amino group.

S40, cross-linking and fully reacting the carboxyl group-activated substituted acridine-polyamino acid conjugate obtained in S30 with a protein to be labelled to obtain an acridine-labelled conjugate.

The resulting acridine-labelled conjugate comprises the substituted acridine, the polyamino acid and the protein to be labelled which are sequentially linked.

The protein to be labelled is a protein, a modified protein, a polypeptide or a modified polypeptide containing an amino group, and the amino group in the protein to be labelled reacts with the carboxyl group in the polyamino acid to form a -NH-CO- structure, thereby linking the polyamino acid and the protein to be labelled together.

The protein to be labelled may be a protein or a polypeptide having an amino group per se, or may be a modified protein or a modified polypeptide into which an amino group is introduced by modification.

In this embodiment, the protein to be labelled is an antigen, a hapten or an antibody.

In cross-linking the carboxyl group-activated substituted acridine-polyamino acid conjugate with the protein to be labelled, a molar ratio of the substituted acridine-polyamino acid conjugate to the protein to be labelled ranges from 5:1 to 1:5.

Preferably, the molar ratio of the substituted acridine-polyamino acid conjugate to the protein to be labelled ranges from 2:1 to 1:2.

The carboxyl group-activated substituted acridine-polyamino acid conjugate is cross-linked with the protein to be labelled to obtain an acridine-labelled conjugate, as shown in following reaction scheme: 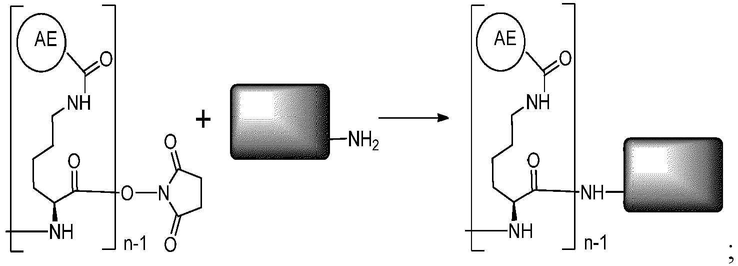 wherein, 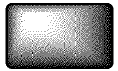 is the protein to be labelled having an amino residue, and n is an integer greater than 25, preferably within a range from 100 to 200.

Specific reaction process of the above reaction scheme is as follows: adding and fully dissolving the carboxyl group-activated substituted acridine-polyamino acid conjugate in a buffer before adding the protein to be labelled and reacting at a temperature ranging from 4°C to 37°C for 0.5 h to 12 h, and after completion of the reaction, purifying to obtain an acridine-labelled conjugate, wherein the buffer is phosphate buffered saline, carbonate buffered saline, 2-(N-morpholino)ethanesulfonic acid (MES) buffer or piperazine-N,N'-bis(2-ethanesulfonic acid) (PIPES) buffer, and the reaction is performed at a pH ranging from 4 to 10.

In such a method for preparing the acridine-labelled conjugate, the polyamino acid reacts, by an amino group therein, with the substituted acridine to form a chemical bond, and the amino group in the protein to be labelled reacts with the carboxyl group in the polyamino acid to form a -NH-CO- structure, thereby linking the polyamino acid and the protein to be labelled together. Due to a relatively identified binding site, the substituted acridine is prevented from interfering with an active site in the protein to be labelled, and thus the acridine-labelled conjugate produced by such method for preparing the acridine-labelled conjugate has relatively high activity.

In addition, the polyamino acid allows an increased steric hindrance of the acridine-labelled conjugate, thereby increasing sensitivity when using the acridine-labelled conjugate.

The present disclosure also discloses a chemiluminescence kit for binding a protein to be labelled to form the acridine-labelled conjugate described above.

The chemiluminescence kit comprises a substituted acridine and a polyamino acid, as defined in the claims.

The polyamino acid is polylysine having a degree of polymerization greater than 25. Preferably, the polyamino acid is polylysine having a degree of polymerization within a range from 100 to 200.

The protein to be labelled is a protein, a modified protein, a polypeptide or a modified polypeptide containing an amino group, and the amino group in the protein to be labelled reacts with the carboxyl group on the polyamino acid to form a -NH-CO- structure, thereby linking the polyamino acid and the protein to be labelled together.

The protein to be labelled may be a protein or a polypeptide having an amino group per se, or may be a modified protein or a modified polypeptide into which an amino group is introduced by modification.

In this embodiment, the protein to be labelled is an antigen, a hapten or an antibody.

Preferably, the chemiluminescent kit further comprises at least one of a centrifugal desalting column and a centrifugal ultrafiltration tube.

In such a chemiluminescent kit, the substituted acridine, the polyamino acid, and the protein to be labelled may be sequentially linked by chemical bonds. Due to a relatively identified binding site, the substituted acridine is prevented from interfering with an active site in the protein to be labelled, and thus the acridine-labelled conjugate formed has relatively high activity.

In addition, the polyamino acid allows an increased steric hindrance of the acridine-labelled conjugate, thereby increasing sensitivity when using the acridine-labelled conjugate.

The following are specific examples.

In this examples, reagents used were purchased from Sigma-aldrich, Inc., and of analytical grade, unless otherwise stated.

Acridinium esters in the examples are all carboxyl-activated acridinium esters. Specific activation is as follows: adding and fully dissolving acridinium ester in phosphate buffered saline (pH 7) before adding EDC and NHS in a molar ration of 1:1, which results in a molar ratio of NHS to acridinium ester of 100:1, and reacting at 25°C for 10 min to obtain an activated substituted acridine.

### Example 1

Polylysine was dissolved in 1 mL of 150 mM PBS buffer (pH 7.4) to a final concentration of 10 nmol/L, and 10 µL of 10 mmol/L acridinium ester in DMSO solvent was added. The mixture was reacted at 25°C for 1 hr and subjected to 5 mL desalting column (7 KD MWCO, Thermo fish) chromatography three times using 150 mM PBS (pH 7.4) as an elution buffer, to remove free acridinium ester and a by-product from the reaction and to obtain an acridine-polylysine solution.

To the above purified acridine-polylysine solution, EDC (at a final concentration of 5 mmol/L) and NHS (at a final concentration of 10 mmol/L) were added. After reaction at 25°C for 30 min, mercaptoethanol was added to a final concentration of 10 mM to obtain activated acridine-polylysine.

To the above activated acridine-polylysine, 1mg of anti-PTH monoclonal antibody (Abnova, Cat.No.: PAB5103, 6.67 nmol) was added, and thoroughly mixed. The mixture was reacted at 25 °C for 1 hr and subjected to 5 mL desalting column (7 KD MWCO, Thermo fish) chromatography three times using 150 mM PBS (pH 7.4) as an elution buffer to remove free NHS and a by-product from the reaction and to obtain a solution of acridinium ester-polylysine-anti-PTH monoclonal antibody conjugate.

### Example 2

Polylysine was dissolved in 1 mL of 150 mM PBS buffer (pH 7.4) to a final concentration of 10 nmol/L, and 10 µL of 10 mmol/L acridinium ester in DMSO solvent was added. The mixture was reacted at 25°C for 1 hr and subjected to 5 mL desalting column (7 KD MWCO, Thermo fish) chromatography three times using 150 mM PBS (pH 7.4) as an elution buffer, to remove free acridinium ester and a by-product from the reaction and to obtain an acridine-polylysine solution.

To the above purified acridine-polylysine solution, EDC (at a final concentration of 5 mmol/L) and NHS (at a final concentration of 10 mmol/L) were added. The mixture was reacted at 25°C for 30 min and subjected to 5 mL desalting column (7 KD MWCO, Thermo fish) chromatography three times using 150 mM PBS (pH 7.4) as an elution buffer, to remove free acridinium ester and a by-product from the reaction and to obtain an acridine-polylysine solution.

To the above acridine-polylysine solution, 1mg of anti-PTH monoclonal antibody (Abnova, Cat.No.: PAB5103, 6.67 nmol) was added, and thoroughly mixed. The mixture was reacted at 25 °C for 1 hr and subjected to 5 mL desalting column (7 KD MWCO, Thermo fish) chromatography three times using 150 mM PBS (pH 7.4) as an elution buffer to remove free NHS and a by-product from the reaction and to obtain a solution of acridinium ester-polylysine-anti-PTH monoclonal antibody conjugate.

### Example 3

Polylysine was dissolved in 1 mL of 150 mM PBS buffer (pH 7.4) to a final concentration of 10 nmol/L, and 10 µL of 10 mmol/L acridinium ester in DMSO solvent was added. The mixture was reacted at 25°C for 1 hr and subjected to ultrafiltration in 4 mL ultrafiltration tube (30 KD MWCO, Millipore) three times using 150 mM PBS (pH 7.4) as an elution buffer, to remove free acridinium ester and a by-product from the reaction and to obtain an acridine-polylysine solution.

To the above purified acridine-polylysine solution, EDC (at a final concentration of 5 mmol/L) and NHS (at a final concentration of 10 mmol/L) were added. The mixture was reacted at 25°C for 30 min and subjected to ultrafiltration in 4 mL ultrafiltration tube (30 KD MWCO, Millipore) three times using 150 mM PBS (pH 7.4) as an elution buffer to obtain activated acridine-polylysine.

To the above activated acridine-polylysine, 1mg of anti-PTH monoclonal antibody (Abnova, Cat.No.: PAB5103, 6.67 nmol) was added, and thoroughly mixed. The mixture was reacted at 25 °C for 1 hr and subjected to ultrafiltration in 4 mL ultrafiltration tube (30 KD MWCO, Millipore) three times using 150 mM PBS (pH 7.4) as an elution buffer to remove free NHS and a by-product from the reaction and to obtain a solution of acridinium ester-polylysine- anti-PTH monoclonal antibody conjugate.

### Comparative Example

1 mg of anti-PTH monoclonal antibody (Abnova, Cat.No.: PAB5103, 6.67 nmol) was dissolved in 1 mL of 150 mM PBS buffer (pH 7.4), and 10 µL of 10 mmol/L acridinium ester in DMSO solvent was added. The mixture was reacted at 25°C for 1 hr and subjected to 5 mL desalting column (7 KD MWCO, Thermo fish) chromatography three times using 150 mM PBS (pH 7.4) as an elution buffer to remove free acridinium ester and a by-product from the reaction and to obtain a solution of acridinium ester- anti-PTH monoclonal antibody.

### Test example 1

The conjugates obtained in Examples 1 to 3 and Comparative Example 1 were used for detecting PTH in a chemiluminescence immunoassay.

To 1000 pg/mL of PTH sample, 50 µg of anti-PTH monoclonal antibody-coated magnetic beads was added, followed by 1 pmol of acridinium ester-labelled anti-PTH antibody prepared by the various methods respectively. After reaction in incubation and washing, 100µL of HNO₃-H₂O₂ solution and 100µL of sodium hydroxide solution were added sequentially, and the resulting solution was measured for a luminescence value using iFlash 3000 chemiluminescence immunoassay analyzer. Measurements were performed in triplicate, and an average value from the measurements was taken as results shown in Table 1. A greater luminescence value from the results in the immunoassay shows the acridinium ester label has a better activity.

**Table 1: Comparison between results from PTH detection using acridinium ester labels prepared by the various methods**

| acridinium ester label | Average luminescence value (RLU) |
|---|---|
| Example 1 | 3124571 |
| Example 2 | 2816943 |
| Example 3 | 2642157 |
| Comparative Example | 752644 |

From the results in Table 1, it can be seen that the acridinium ester-labelled reagents prepared in Examples 1 to 3 exhibited significantly better activity than that prepared in Comparative Example.

### Test example 2

The solution of acridinium ester-polylysine anti-PTH monoclonal antibody obtained in Example 1 was used for detecting PTH having a serial of concentrations in chemiluminescence immunoassay. To respective PTH sample solutions having a serial of concentrations, 50 µg of anti-PTH monoclonal antibody-coated magnetic bead reagent and 1 pmol acridinium ester-polylysine-anti-PTH monoclonal antibody reagent were added. After reaction in incubation and washing, 100µL of HNO₃-H₂O₂ solution and 100µL sodium hydroxide solution were added sequentially, and the resulting solution was measured for a luminescence value using iFlash 3000chemiluminescence immunoassay analyzer. Results are shown in Table 2, and data from Table 2 is plotted with the concentration of PTH antigen on the X-axis against the relative luminescence value plotted on the Y-axis, as shown in Fig.2.

**Table 2: Immunoassay results for PTH antigen samples having a serial of concentrations**

| PTH antigen concentration | Luminescence value (RLU) |
|---|---|
| 0pg/mL | 1528 |
| 13.91pg/mL | 33615 |
| 25.74pg/mL | 121932 |
| 140.79pg/mL | 602764 |
| 696.54pg/mL | 2848273 |
| 3701.6pg/mL | 14004950 |

From the results in Table 2 and Fig. 2, it can be known that the acridinium ester -polylysine-antibody prepared in Example 1 can be applied in chemiluminescence immunoassay and has a good effect. The method for preparing the acridinium ester-polylysine-label according to Example 1 has good applicability.

## Claims

1. An acridine-labelled conjugate, comprising a substituted acridine, a polyamino acid, and a protein to be labelled which are sequentially linked;
the polyamino acid is a polyamino acid containing a carboxyl group and an amino group, and the polyamino acid reacts, by the amino group therein, with the substituted acridine to form a chemical bond;
the protein to be labelled is a protein, a modified protein, a polypeptide or a modified polypeptide containing an amino group, and the amino group in the protein to be labelled reacts with the carboxyl group in the polyamino acid to form a -NH-CO- structure, thereby linking the polyamino acid and the protein to be labelled together; and
the substituted acridine is 10-methyl-acridine-9-N-succinimidyl ester formic acid (AE-NHS) and the polyamino acid is polylysine.

2. The acridine-labelled conjugate according to claim 1, wherein the polyamino acid is a polyamino acid having a degree of polymerization greater than 25.

3. The acridine-labelled conjugate according to claim 1 or 2, wherein the protein to be labelled is an antigen, a hapten or an antibody.

4. A method for preparing the acridine-labelled conjugate according to any one of claims 1 to 3, comprising:
covalently cross-linking and fully reacting an activated substituted acridine with a polyamino acid to obtain a substituted acridine-polyamino acid conjugate, wherein the polyamino acid is a polyamino acid containing a carboxyl group and an amino group, and the polyamino acid reacts, by an amino group therein, with substituted acridine to form a chemical bond ;
purifying the substituted acridine-polyamino acid conjugate;
activating the carboxyl group in a purified substituted acridine-polyamino acid conjugate by using a crosslinking agent;
cross-linking and fully reacting a carboxyl group-activated substituted acridine-polyamino acid conjugate with a protein to be labelled to obtain the acridine-labelled conjugate, wherein the acridine-labelled conjugate comprises a substituted acridine, a polyamino acid and the protein to be labelled which are sequentially linked, the protein to be labelled is a protein, a modified protein, a polypeptide or a modified polypeptide containing an amino group, and the amino group in the protein to be labelled reacts with the carboxyl group in polyamino acid to form a -NH-CO- structure, thereby linking polyamino acid and the protein to be labelled together; and
the substituted acridine is AE-NHS and the polyamino acid is polylysine.

5. The method according to claim 4, wherein a molar ratio of activated substituted acridine to polyamino acid ranges from 1:1 to 10000:1 in covalently crosslinking activated substituted acridine with polyamino acid.

6. The method according to claim 4, wherein a molar ratio of the carboxyl group-activated substituted acridine-polyamino acid conjugate to the protein to be labelled ranges from 5:1 to 1:5 in cross-linking the carboxyl group-activated substituted acridine-polyamino acid conjugate with a protein to be labelled.

7. The method according to claim 4, wherein the crosslinking agent comprises carbodiimide and hydroxysuccinimide in activating the carboxyl group in the purified substituted acridine-polyamino acid conjugate by using the crosslinking agent.

8. The method according to claim 7, wherein: carbodiimide is at least one selected from dicyclohexylcarbodiimide, 1-(3-dimethylamino propyl)-3-ethyl carbodiimide, and N,N'-diisopropyl carbodiimide, and a molar ratio of carbodiimide to the substituted acridine-polyamino acid conjugate ranges from 5:1 to 1000:1; and hydroxysuccinimide is at least one selected from N-hydroxysuccinimide and N-hydroxysulfosuccinimide, and a molar ratio of carbodiimide to hydroxysuccinimide ranges from 10:1 to 1:20.

9. A chemiluminescence kit for binding a protein to be labelled to form the acridine-labelled conjugate according to any one of claims 1 to 3, comprising a substituted acridine and a polyamino acid;
the polyamino acid is a polyamino acid containing a carboxyl group and an amino group, and the polyamino acid reacts, by the amino group therein, with the substituted acridine to form a chemical bond;
the protein to be labelled is a protein, a modified protein, a polypeptide or a modified polypeptide containing an amino group, and the amino group in the protein to be labelled reacts with the carboxyl group in the polyamino acid to form a -NH-CO- structure, thereby linking polyamino acid and the protein to be labelled together; and
the substituted acridine is AE-NHS and the polyamino acid is polylysine.

## Patentansprüche

1. Acridin-markiertes Konjugat, das ein substituiertes Acridin, eine Polyaminosäure und ein zu markierendes Protein umfasst, die sequenziell aneinander gebunden sind;
wobei die Polyaminosäure eine Polyaminosäure ist, die eine Carboxylgruppe und eine Aminogruppe enthält, und die Polyaminosäure durch die Aminogruppe darin mit dem substituierten Acridin reagiert, um eine chemische Bindung zu bilden;
das zu markierende Protein ein Protein, ein modifiziertes Protein, ein Polypeptid oder ein modifiziertes Polypeptid, ein Polypeptid oder ein modifiziertes Polypeptid, das eine Aminogruppe enthält, ist und die Aminogruppe in dem zu markierenden Protein mit der Carboxylgruppe in der Polyaminosäure reagiert, um eine -NH-CO--Struktur zu bilden, wodurch die Polyaminosäure und das zu markierende Protein aneinander gebunden werden; und
das substituierte Acridin 10-Methylacridin-9-N-succinimidylameisensäureester (AE-NHS) ist und die Polyaminosäure Polylysin ist.

2. Acridin-markiertes Konjugat nach Anspruch 1, wobei die Polyaminosäure eine Polyaminosäure ist, die einen Polymerisationsgrad von mehr als 25 aufweist.

3. Acridin-markiertes Konjugat nach Anspruch 1 oder 2, wobei das zu markierende Protein ein Antigen, ein Hapten oder ein Antikörper ist.

4. Verfahren zur Herstellung eines Acridin-markierten Konjugats nach einem der Ansprüche 1 bis 3, wobei das Verfahren Folgendes umfasst:
das kovalente Vernetzen und vollständige Umsetzen eines aktivierten substituierten Acridins mit einer Polyaminosäure zum Erhalt eines substituierten Acridin-Polyaminosäure-Konjugats, wobei die Polyaminosäure eine Polyaminosäure ist, die eine Carboxylgruppe und eine Aminogruppe enthält, und die Polyaminosäure durch die Aminogruppe darin mit substituiertem Acridin reagiert, um eine chemische Bindung zu bilden;
das Reinigen des substituierten Acridin-Polyaminosäure-Konjugats;
das Aktivieren der Carboxylgruppe in einem gereinigten substituierten Acridin-Polyaminosäure-Konjugat unter Verwendung eines Vernetzungsmittels;
das Vernetzen und vollständige Umsetzen eines Carboxylgruppe-aktivierten substituierten Acridin-Polyaminosäure-Konjugats mit einem zu markierenden Protein zum Erhalt des Acridin-markierten Konjugats, wobei das Acridin-markierte Konjugat ein substituiertes Acridin, eine Polyaminosäure und das zu markierende Protein umfasst, die sequenziell aneinander gebunden sind, wobei das zu markierende Protein ein Protein, ein modifiziertes Protein, ein Polypeptid oder ein modifiziertes Polypeptid, das eine Aminogruppe enthält, ist und wobei die Aminogruppe in dem zu markierenden Protein mit der Carboxylgruppe in der Polyaminosäure reagiert, um eine -NH-CO--Struktur zu bilden, wodurch Polyaminosäure und das zu markierende Protein aneinander gebunden werden; und
wobei das substituierte Acridin AE-NHS ist und die Polyaminosäure Polylysin ist.

5. Verfahren nach Anspruch 4, wobei ein Molverhältnis von aktiviertem substituiertem Acridin zu Polyaminosäure beim kovalenten Vernetzen von aktiviertem substituiertem Acridin mit Polyaminosäure im Bereich von 1:1 bis 10.000:1 liegt.

6. Verfahren nach Anspruch 4, wobei ein Molverhältnis des Carboxylgruppen-aktivierten substituierten Acridin-Polyaminosäure-Konjugats zu dem zu markierenden Protein beim Vernetzen des Carboxylgruppen-aktivierten substituierten Acridin-Polyaminosäurekonjugats mit einem zu markierenden Protein im Bereich von 5:1 bis 1:5 liegt.

7. Verfahren nach Anspruch 4, wobei das Vernetzungsmittel Carbodiimid und Hydroxysuccinimid beim Aktivieren der Carboxylgruppe in dem gereinigten substituierten Acridin-Polyaminosäure-Konjugat unter Verwendung des Vernetzungsmittels umfasst.

8. Verfahren nach Anspruch 7, wobei: Carbodiimid zumindest eines ausgewählt aus Dicyclohexylcarbodiimid, 1-(3-Dimethylaminopropyl)-3-ethylcarbodiimid und N,N'-Diisopropylcarbodiimid ist und ein Molverhältnis von Carbodiimid zu dem substituierten Acridin-Polyaminosäure-Konjugat im Bereich von 5:1 zu 1.000:1 liegt und Hydroxysuccinimid zumindest eines ausgewählt aus N-Hydroxysuccinimid und N-Hydroxysulfosuccinimid ist und ein Molverhältnis von Carbodiimid zu Hydroxysuccinimid im Bereich von 10:1 bis 1:20 liegt.

9. Chemilumineszenz-Set zur Bindung eines zu markierenden Proteins zur Bildung eines Acridin-markierten Konjugats nach einem der Ansprüche 1 bis 3, das ein substituiertes Acridin und eine Polyaminosäure umfasst;
wobei die Polyaminosäure eine Polyaminosäure ist, die eine Carboxylgruppe und eine Aminogruppe enthält, und die Polyaminosäure durch die Aminogruppe darin mit dem substituierten Acridin reagiert, um eine chemische Bindung zu bilden;
das zu markierende Protein ein Protein, ein modifiziertes Protein, ein Polypeptid oder ein modifiziertes Polypeptid, das eine Aminogruppe enthält, ist und die Aminogruppe in dem zu markierenden Protein mit der Carboxylgruppe in der Polyaminosäure reagiert, um eine - NH-CO--Struktur zu bilden, wodurch Polyaminosäure und das zu markierende Protein aneinander gebunden werden; und
das substituierte Acridin AE-NHS ist und die Polyaminosäure Polylysin ist.

## Revendications

1. Conjugué marqué à l'acridine, comprenant une acridine substituée, un polyaminoacide et une protéine à marquer qui sont liés séquentiellement ;
Le polyaminoacide est un polyaminoacide contenant un groupe carboxyle et un groupe amino, et le polyaminoacide réagit, via le groupe amino en son sein, avec l'acridine substituée pour former une liaison chimique ;
la protéine à marquer est une protéine, une protéine modifiée, un polypeptide ou un polypeptide modifié contenant un groupe amino, et le groupe amino dans la protéine à marquer réagit avec le groupe carboxyle dans le polyaminoacide pour former une structure -NH-CO-, en liant ainsi le polyaminoacide et la protéine à marquer ensemble ; et
l'acridine substituée est de l'ester 10-méthyl-acridine-9-N-succinimidylique d'acide formique (AE-NHS) et le polyaminoacide est de la polylysine.

2. Conjugué marqué à l'acridine selon la revendication 1, dans lequel le polyaminoacide est un polyaminoacide ayant un degré de polymérisation supérieur à 25.

3. Conjugué marqué à l'acridine selon la revendication 1 ou 2, dans lequel la protéine à marquer est un antigène, un haptène ou un anticorps.

4. Procédé de préparation du conjugué marqué à l'acridine selon l'une quelconque des revendications 1 à 3, comprenant les étapes consistant à :
réticuler de manière covalente et faire réagir complètement une acridine substituée activée avec un polyaminoacide pour obtenir un conjugué acridine substituée-polyaminoacide, dans lequel le polyaminoacide est un polyaminoacide contenant un groupe carboxyle et un groupe amino, et le polyaminoacide réagit, via un groupe amino en son sein, avec l'acridine substituée pour former une liaison chimique ;
purifier le conjugué acridine substituée-polyaminoacide,
activer le groupe carboxyle dans un conjugué acridine substituée-polyaminoacide purifié en utilisant un agent de réticulation ;
réticuler et faire réagir complètement un conjugué acridine substituée activée par un groupe carboxyle - polyaminoacide avec une protéine à marquer pour obtenir le conjugué marqué à l'acridine, dans lequel le conjugué marqué à l'acridine comprend une acridine substituée, un polyaminoacide et la protéine à marquer qui sont liés séquentiellement, la protéine à marquer est une protéine, une protéine modifiée, un polypeptide ou un polypeptide modifié contenant un groupe amino, et le groupe amino dans la protéine à marquer réagit avec le groupe carboxyle dans le polyaminoacide pour former une structure -NH-CO-, en liant ainsi le polyaminoacide et la protéine à marquer ensemble ; et
l'acridine substituée est AE-NHS et le polyaminoacide est de la polylysine.

5. Procédé selon la revendication 4, dans lequel un rapport molaire de l'acridine substituée activée sur le polyaminoacide est compris entre 1:1 et 10 000:1 dans la réticulation covalente de l'acridine substituée activée avec un polyaminoacide.

6. Procédé selon la revendication 4, dans lequel un rapport molaire du conjugué acridine substituée activée par un groupe carboxyle-polyaminoacide à la protéine à marquer est compris entre 5:1 et 1:5 dans la réticulation du conjugué acridine substituée activée par un groupe carboxyle-polyaminoacide avec une protéine à marquer.

7. Procédé selon la revendication 4, dans lequel l'agent de réticulation comprend un carbodiimide et un hydroxysuccinimide en activant le groupe carboxyle dans le conjugué acridine substituée-polyaminoacide purifié en utilisant l'agent de réticulation.

8. Procédé selon la revendication 7, dans lequel : le carbodiimide est au moins un choisi parmi le dicyclohexylcarbodiimide, le 1-(3-diméthylamine propyl)-3-éthylcarbodiimide et le N,N'-diisopropylcarbodiimide, et le rapport molaire du carbodiimide au conjugué acridine substituée-polyaminoacide est compris entre 5:1 et 1 000:1 ; et l'hydroxysuccinimide est au moins un choisi parmi le N-hydroxysuccinimide et le N-hydroxysulfosuccinimide, et le rapport molaire du carbodiimide à l'hydroxysuccinimide est compris entre 10:1 et 1:20.

9. Kit de chimioluminescence pour lier une protéine à marquer pour former le conjugué marqué à l'acridine selon l'une quelconque des revendications 1 à 3, comprenant une acridine substituée et un polyaminoacide ;
le polyaminoacide est un polyaminoacide contenant un groupe carboxyle et un groupe amino, et le polyaminoacide réagit, via le groupe amino en son sein, avec l'acridine substituée pour former une liaison chimique ;
la protéine à marquer est une protéine, une protéine modifiée, un polypeptide ou un polypeptide modifié contenant un groupe amino, et le groupe amino dans la protéine à marquer réagit avec le groupe carboxyle dans le polyaminoacide pour former une structure -NH-CO-, en liant ainsi le polyaminoacide et la protéine à marquer ensemble ; et
l'acridine substituée est AE-NHS et le polyaminoacide est de la polylysine.
